# EUROPEAN PATENT SPECIFICATION

(11) **EP 1 802 248 B1**
(45) Date of publication and mention of the grant of the patent: **08.06.2011**
(21) Application number: 05857641.4
(22) Date of filing: 14.10.2005
(51) Int. Cl.: A61C 7/08, A63B 71/08

(54) **ORAL APPLIANCE**
Orale Vorrichtung
APPAREIL ORAL

(30) Priority: 14.10.2004 AU 2004905924
(43) Date of publication of application: 04.07.2007
(73) Proprietor: Farrell, Christopher John, Helensvale, Queensland 4212 (AU)
(72) Inventor: Farrell, Christopher John, Helensvale, Queensland 4212 (AU)
(74) Representative: Olesen, Kaj
(86) International application number: PCT/AU2005/001598
(87) International publication number: WO 2006/108209

(56) References cited:
- WO-A-00/35369
- US-A- 2 827 899
- US-A- 3 224 443
- US-A- 5 031 638
- US-A- 5 082 007
- US-A- 5 511 562
- US-A1- 2003 101 999
- US-A1- 2004 103 905
- US-A1- 2004 154 625
- US-A1- 2004 154 626
- US-A1- 2004 154 626

## Description

### Field of the Invention

This invention relates to an oral appliance.

This invention relates particularly but not exclusively to an oral appliance that is an orthodontic appliance used to assist in correcting the misalignment of teeth by applying a correcting force to the teeth. It will therefore be convenient to hereinafter describe this invention in detail with reference to this example application. However it is to be clearly understood that the invention is capable of broader application. For example the invention might be applied to appliances that encourage an improvement in oral habits such as tongue thrusting without applying a repositioning force to the teeth.

Finally the appliance could also be used as a mouth guard or sports guard as well.

### Background to the Invention

Various types of orthodontic appliances are known. One such type of orthodontic appliance is custom made after taking impressions of a patient's mouth. The impressions are used to make moulds which in turn are used to make an appliance that is specifically dimensioned and configured to suit that patient's mouth. Not surprising these appliances fit snugly into a user's mouth and are efficacious in operation. However the limitation of custom made appliances is the high cost of producing such an appliance and this has reduced their uptake in the market place.

There is therefore a clear need for an appliance that can be manufactured in large quantities in a manufacturing process and that can also be fitted to fit a variety of patient's mouths with a comfortable and snug fit.

One mass produced orthodontic trainer is produced by Myofunctional Research Pty Ltd, of Helensvale Plaza, Sir John Overall Drive, Helensvale, in the state of Queensland, Australia.

The trainer has a shape resembling that of an arch and defines upper and lower channels for receiving respectively the teeth of the upper and lower arches of a user.

The trainer is made of silicone rubber and is moulded in a single moulding operation. Thus the silicon rubber has a single homogeneous form and consistency throughout ' the appliance and is generally soft and flexible. The walls of the trainer that define channels for the upper and lower teeth are soft and therefore comfortable against the teeth and gums of a user. However because the trainer is soft it does not apply a strong retaining and correcting force to teeth that are received within the channels. For example it is easy to bend and twist the member.

The trainer described above has been used to try and improve the oral habits of users, in particular children. The tongue tab assists in correctly positioning the tongue and reducing tongue thrusting. It also correctly positions the lower jaw with respect to the upper jaw.

The trainer has also been used to try and improve the alignment of teeth and has been found to have some positive influence on correcting the misalignment of teeth. However there is still room for considerable improvement in this regard. The softness of the silicone rubber material that abuts the teeth really limits the ability of the trainer to reposition a user's teeth.

One possibility would appear to be to make the trainer from a material that was harder than silicone rubber such as polyurethane. However the polyurethane would be hard against the soft gums of the user and cause pain and discomfort, In addition if the appliance was made of a single hard material it would have limited ability to bend and deform to receive misaligned teeth. It would thus be harder to fit to a wide number of users all with their own individual arch sizes and teeth alignments. Thus there would be difficulties in fitting the trainer in the first place.

WO-A-00 35 368 describes an oral appliance usable either as a mouthguard or an orthodontic device. The oral appliance includes a base member having a U-shaped form corresponding to a row of teeth of a user. The base member has inner and outer flanges interconnected by a web which collectively define upper and lower channels, within which the upper and lower rows of teeth of the user are received. Upper and lower teeth engaging elements are positioned within the upper and lower channels. The teeth engaging elements are made of EVA which softens at a temperature of 90 to 95°C and are moulded to the teeth of the user. The base member remains unaffected by heating to 90 to 95°C. The oral appliance may also include a tongue tag and breathing apertures.

While it is evident that there is no simple solution to this problem clearly it would be extremely useful if a trainer could be devised that was soft and comfortable to wear and which also generated a strong repositioning force. This would achieve the benefits of the prior art silicone rubber trainer while also exerting a stronger influence on the realignment of a user's teeth. Such a trainer would provide a great step or leap forward in the art of orthodontic trainers.

### Summary of the Invention

According to one aspect of this invention there is provided an oral appliance, that is an orthodontic trainer, comprising: a base member having a generally U-shaped form having left and right arms, corresponding to the outline of the jaw of a user, the base member being made of a polymeric material that is resilient and is capable of some bending when opposing arms of the base member are pulled towards and away from each other; and
a continuous teething engaging member made of a material that is softer than the material of the base member that encloses and encases at least part of the base member and defines at least one of upper and lower teeth channels within which the associated arch and teeth of the user can be received.

The base member is designed with a wide arch so that when fitted to a user having a narrow arch, the arch is urged to widen by the resilient force of the deformed base member.

The oral appliance is an orthodontic trainer for assisting in maintaining the user's teeth in a desired position and/or promoting movement of the teeth into a more desirable position.

The oral appliance may be a sports guard or a mouthguard for providing protection for a user's teeth when playing sports such as contact sports and the like.

The teeth engaging member may be made of a polymeric material.

The polymeric material may be a polymeric material containing silicon as a repeating unit within the polymer. This may be a synthetic elastomer which is a cross linked polymer reinforced with silica.

The polymeric material may be a siloxane polymer or a silane polymer. In one form of the invention the polymer containing silicon is silicone rubber, e.g. medical grade silicone which has already found acceptance in the field of orthodontic trainers. The silicone material will be particularly soft and comfortable against the gums and other mouth tissues of a user. It has some ability to deform and bend around the teeth and gums.

Alternatively the polymeric material of the teeth engaging member may be an addition polymer. The addition polymer may be polyvinylchloride (PVC). Polyvinyl chloride has a melting point that is lower than that for silicone. As a result the temperature to which the base member is exposed to when the teeth engaging member is moulded onto it is lower when polyvinyl chloride is used for the teeth engaging member than when silicone rubber is used.

The polymeric material from which the teeth engaging member is formed may be categorised as a hard material. The material from which the base member is made is characterised by the fact that it does not deform easily when local pressure or point pressure is applied to the base member. However at the same time it is capable of some bending when opposing sides of the member are twisted or pulled towards and away from each other.

Further the polymeric material from which the base member is made may have thermoplastic properties. However the material may maintain its form and not soften when it is exposed to flowing material at a temperature of 160 degrees Celsius or below. In some embodiments it may maintain its form and not soften when exposed to material at a temperature of up to 300°C.

The polymeric material from which the base member is made may be a polyamide material. The polyamide material may be formed either by a condensation polymerisation of amide monomers or a ring opening polymerisation of caprolactam.

The polyamide material may be a polyamide polymer commonly known as nylon or sold under the trade mark NYLON which is the trade mark of the Du Pont Chemical company, Wilmington, Delaware, U.S.A.

The polyamide material, e.g. nylon, is particularly suitable because it has a suitable balance between resilience, and stiffness. It does permit some flexing of the arms of the base member. However it has a good memory when subjected to bending or twisting forces and tends to return to its original form when the forces are removed. This helps the base member to maintain the desired arch while still maintaining a reasonable degree of wearer comfort.

Alternatively the polymeric material of the base member may be an addition polymer or a condensation polymer. The addition polymer may include polyethylene or polypropylene. The condensation polymer may include polyurethane or polycarbonate. The polymeric material may also include a thermoplastic elastomer such as santoprene.

In one embodiment of the invention, the base member is made of polyamide material and the teeth engaging member is made of silicone rubber.

With this combination the polyamide, e.g. nylon, base member is moulded first during the manufacture of the trainer. Thereafter the silicone teeth engaging member is moulded onto the base member. The nylon base member has the property that it does not soften and melt when it is exposed to molten silicone rubber when the silicone rubber is injected into the mould and encases the base member.

Thus the oral appliance essentially comprises a base member that is formed from a material that provides an underlying skeletal strength to the appliance, and an encasing teeth engaging member of silicone rubber or PVC that is considerably softer and more formable than the base member. By having a rigid underlying base member the appliance is able to exert a considerably stronger influence on the alignment of a patient's teeth than if the appliance were made solely of soft silicone rubber. In addition it is able to preserve the general shape and the width of the arch.

The base member may be in the form of an open frame structure, eg a flat open frame structure, with curved inner and outer longitudinal frame members that are interconnected at spaced intervals by transverse frame members. The open frame structure may lie broadly in a plane. That is the longitudinal frame members and the transverse frame members may all lie broadly in the same plane. The plane may extend in a substantially horizontally extending direction.

The base member may further include an outer teeth repositioning formation on the outer longitudinal frame member.

The outer teeth repositioning formation may comprise an outer flange projecting up from the outer frame member above the height of the transverse frame members and/or depending down from the outer frame member below the level of the transverse frame members.

The base member may further include an inner teeth repositioning formation on the inner longitudinal frame member.

The inner teeth repositioning formation may comprise an inner flange projecting up from the inner frame member above the height of the transverse frame members and/or depending down from the inner frame member below the level of the transverse frame members.

The base member may have a central front region and left and right arm regions extending rearward from the central front region to left and right rear ends.

The outer flange may project upwardly from the transverse frame members along at least said central front region. Thus in use the outer flange extends over and across the upper front teeth of a user.

The central front region of the outer flange, may extend upwardly above the transverse frame members, e.g. an upper surface of the transverse frame members, by 2-10mm, e.g. 4-8mm, e.g. about 6mm. The central front region of the outer flange may also extend above the plane of the open frame by 2-10mm, e.g. 4-8mm, e.g. about 6mm. Thus the outer flange may have some height, particularly in the central front regions.

The outer flange may also project upwardly above the transverse frame members along left and right arm regions of the base member.

The left and right arm regions of the outer flange may extend upwardly above the transverse frame members, e.g. an upper surface of the transverse frame members, by 2-6mm, e.g. 3-5mm, e.g. about 4mm. The left and right arm regions of the outer flange may also extend upwardly above the plane of the open frame by 2-6mm, e.g. 3-5mm, e.g. about 4mm. Thus the outer flange in the central front region is higher than the flange on the left and right arm regions, e.g. which extends over at least part of the molars of the user.

Further the outer flange may be interrupted, or be of reduced height, at positions on the left and right hand sides intermediate the left and right arm regions of the outer flange and the central front region of the outer flange. These positions on the left and right sides correspond to the canine teeth. The height of the outer flange is reduced at these points because the canine teeth sometimes project out relative to the other teeth and may not fit behind the flange if the outer flange was the same height as it is in the central front region.

The outer flange may be formed integrally with the curved outer longitudinal frame member, e.g. in an injection moulding operation.

The inner flange on the curved inner longitudinal frame member may extend a distance of about 1-3mm, e.g. about 2mm up from the transverse frame members, e.g. the upper surface of the transverse frame members. The inner flange on the curved inner longitudinal frame member may extend a distance of about 1-3mm, e.g. about 2mm up from the plane of the open frame.

The inner flange may project up from the transverse frame members along at least the central front region of the inner longitudinal frame member. The inner flange may project up from the transverse frame members along the full length of the inner longitudinal frame member. Further the inner flange may project up to substantially the same height along its full length.

The inner flange may also be formed integrally with the curved inner element, eg in an injection moulding process.

In one form of the invention the outer flange on the curved outer longitudinal frame member does not extend or depend downwardly below the plane of the open frame to any appreciable extent. Similarly the flange on the curved inner longitudinal frame member does not extend or depend downwardly below the plane of the open frame to any appreciable extent.

However it needs to be appreciated that an appliance with one or more downwardly depending flanges is contemplated to be within the scope of the invention. Applicant has found that a satisfactory stiffness and strength can be obtained with outer and inner flanges that only project up from the open frame and not down from the open frame. However if greater strength was required near the outer longitudinal frame member, e.g. on the outside of the teeth, the flange could depend downwardly as well as projecting upwardly. A downwardly depending inner flange could also be provided.

In a further alternative the inner and outer flanges described above could also be provided projecting down from the plane of the open frame or the transverse frame members instead of projecting up from the transverse frame members.

The base member may have a front said transverse frame member ih the central front region of the base member, and two rear said transverse frame members at the rear of the left and right arm regions of the base member.

The base member may also have a plurality of intermediate said transverse frame members in between the rear transverse frame members at the rear of the arm regions and the front transverse frame member.

The transverse frame members in the central front region of the base member may have a width of 5-15mm, preferably 8-12mm, and most preferably about 9-11mm. The rear transverse frame members at the rear of each of the arm regions may have a width of 2-10mm, preferably 3-8mm, more preferably 4-6mm. The intermediate transverse frame members may have a width of 1-4mm, e.g. 2-3mm.

Thus the transverse frame member disposed in the front region of the base member, eg substantially centrally, may have a greater width than the rear and intermediate frame members. These transverse frame members or cross elements perform important structural functions and strengthen the appliance in key areas such as the front and the rear of the arm regions.

The teeth engaging member may fully encase the open frame structure of the base member, e.g. including both upper and lower faces of the open frame structure, and also inner and outer sides of the open frame structure.

The teeth engaging member may comprise a central web having upper and lower surfaces that extends parallel- to the plane of the open frame, and also inner and outer flanges that project transversely away from at least one of the upper and lower surfaces of the web. The inner and outer flanges and the web may form at least one teeth engaging formation defining a channel within which the teeth of a user are received.

The web and inner and outer flanges may form both upper and lower teeth engaging formations in which case the flanges of the member project away from both the upper and lower surfaces of the web. These teeth engaging formations formed by the flanges and the web define said upper and lower channels of the teeth engaging member.

The teeth engaging member extends around and over the base member and forms the body of the trainer. It defines the surfaces of the trainer that come into contact with and engage the teeth and gums of the user.

The teeth engaging member may encase the full surface of the base member, eg including an outwardly facing surface of the outer flange. The teeth engaging member may cover an outer surface of the outer flange with a relatively thin layer of material, e.g. substantially thinner than that covering an inside surface of the outer flange. This assists in holding the base member and teeth engaging member together.

The advantage of the teeth engaging member encasing the base member is that it assists in holding the teeth engaging member onto the base member. The base member and the teeth engaging member are made of different materials and the different materials need to conform and move with each other so that they do not delaminate. The encasement of the base member within the teeth engaging member assists in holding the two together and the appliance as a result is resistant to delamination in use.

The teeth engaging member may have broadly the same shape and form as that illustrated in the Applicant's earlier patent application PCT/AU99/00840 and published by WIPO as WO 00/35369.

The teeth engaging member may further include one or more teeth positioning formations in at least one of the channels for assisting in positioning the individual teeth of a user, e.g. a plurality of said teeth positioning formations. The positioning formations may also assist in fitting the appliance initially.

The-teeth positioning formations may be arranged in pairs, that are aligned with each other along the length of the channel or arch that project inward from the inner and outer flanges respectively.

Each teeth positioning formation may comprise a wedge shaped protrusion extending inwardly from a said flange into a said channel. Each positioning formation may be broadly shaped to be complementary to the shape of the user's teeth and may extend into the associated channel only a small distance.

The teeth positioning formations may be positioned so as to encourage the teeth to move towards so called ideal positions for these teeth within the mouth of a user.

The teeth positioning formations may collectively position at least the central front two incisors of the teeth of a user, e.g. at least the front four teeth of a user. In one form the teeth positioning formations collectively position the front four teeth of a user. In one form the teeth positioning formations collectively position the front four teeth of a user and the three teeth on each side rearward of the front four teeth of a user.

The protrusions may be integrally moulded with the teeth engaging member, e.g. by injection moulding, when the teeth engaging member is moulded onto base member.

As indicated above, in a preferred form the teeth engaging member defines both upper and lower channels for receiving the upper and lower teeth of a user. In such a trainer the teeth engaging member may have teeth positioning formations in both said upper and lower channels for positioning both the upper teeth and the lower teeth.

The orthodontic trainer may include holes passing through the teeth engaging member and base member and opening to both the outer surfaces of the teeth engaging member and the inner surface thereof.

The orthodontic trainer may also include a tongue tab for encouraging a user to correctly position their tongue.

The orthodontic trainer may also include a cutaway or recess defined thereof in the front region of the teeth engaging member, e.g. the curved inner flange, for enabling the width of the arch defined by the trainer, e.g. particularly the left and right arm regions of the teeth engaging and base member, to be adjusted. Conveniently this may be provided by the spaces on each side of the tongue tab.

The orthodontic trainer may also include a cut away on the midline of the curved outer flange, e.g. both above and below the web. This is provided so that this soft gum region does not make contact with the teeth engaging member when the trainer is worn to enhance comfort.

The appliance may also have some thickening of the web of the teeth engaging member in the left and right arm regions thereof. The thickness of the teeth engaging member may increase in a direction rearward from a central front region towards the rear of the left and right arm regions. This thickening may terminate at a point spaced forwardly of the rear of the left and right arm regions of the teeth engaging member. The teeth engaging member may then thin again from this point to the rear of the left and right arm regions.

The thickening of the teeth engaging member may resemble an inverted aerofoil, e.g. with a curved lower surface and a substantially planar upper surface, when viewed in cross section. This shape of the web region of the teeth engaging member correctly positions the teeth of the upper and lower jaw relative to each other so as to encourage relaxation of the muscles and also the TMJ.

Applicant envisages that the appliance will be made in several sizes and a suitable size of trainer selected for any given user based on the size of their teeth. Applicant envisages that the different sizes will comprise two different arch sizes and each arch size will have about six different sizes and positions of teeth locating formations. The different teeth locating formations will accommodate the different teeth sizes and teeth positions of different users.

### Detailed Description of the Preferred Embodiments

An oral appliance that is an orthodontic trainer in accordance with this=invention -may manifest itself in a variety of forms. It will be convenient to hereinafter provide a detailed description of some embodiments of the invention with reference to the accompanying drawings. In the drawings:
Fig 1 is an upper front three dimensional view of an appliance that is an orthodontic trainer in accordance with the invention;
Fig 2 is an upper rear three dimensional view of the appliance of Fig 1;
Fig 3 is a front view of the appliance of Fig 1;
Fig 4 is a rear view of the appliance of Fig 1;
Fig 5 is a top plan-view of the appliance of Fig 1;
Fig 6 is a bottom plan view of the appliance of Fig 1;
Fig 7 is a side view of the appliance of Fig 1 from one side;
Fig 8 is a side view of the appliance of Fig 1 from the other side;
Fig 9 is an upper three dimensional view of part of the appliance of Fig 1 with part of the teeth engaging member removed to expose an underlying.base member;
Fig 10 is an upper three dimensional view of a base member for the appliance of Fig 1; and
Fig 11 is a lower three dimensional view of the base member of Fig 10.

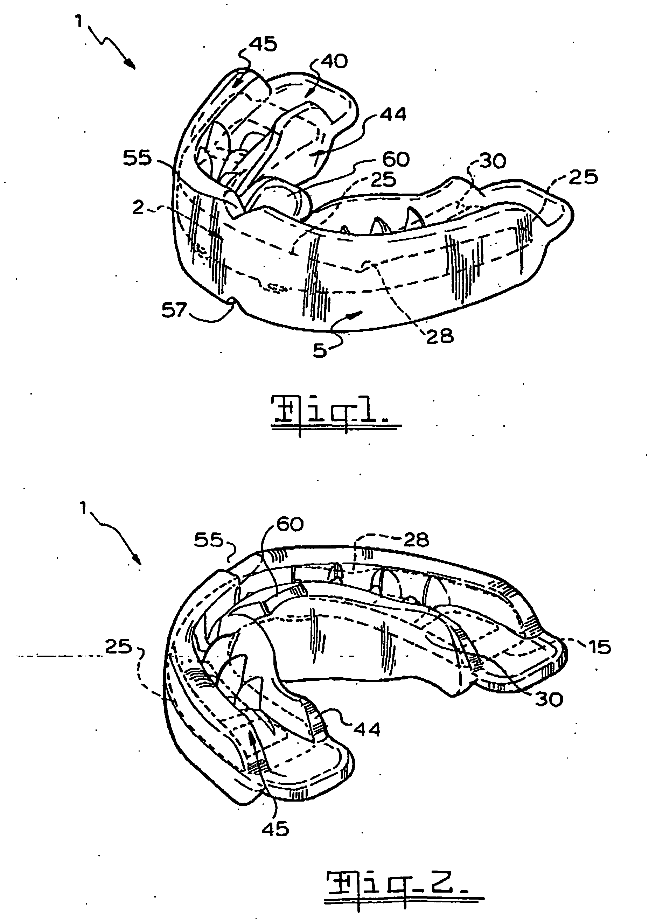

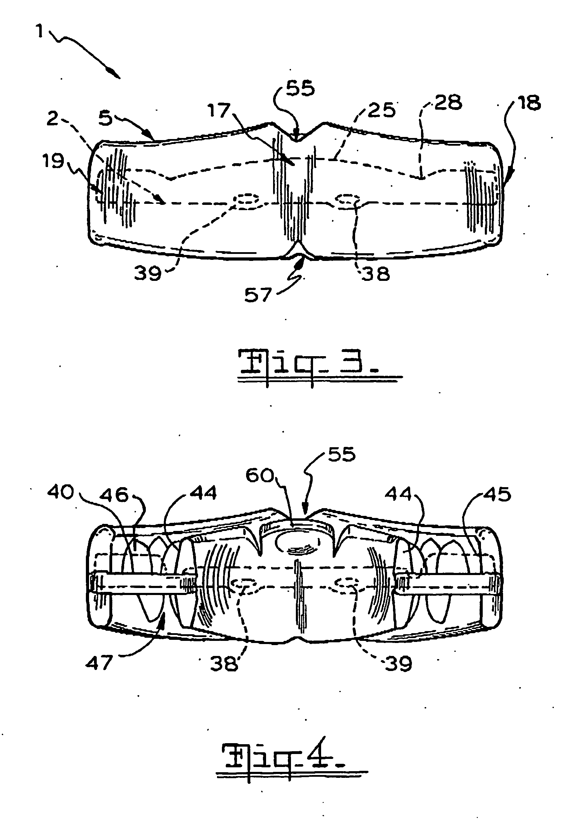

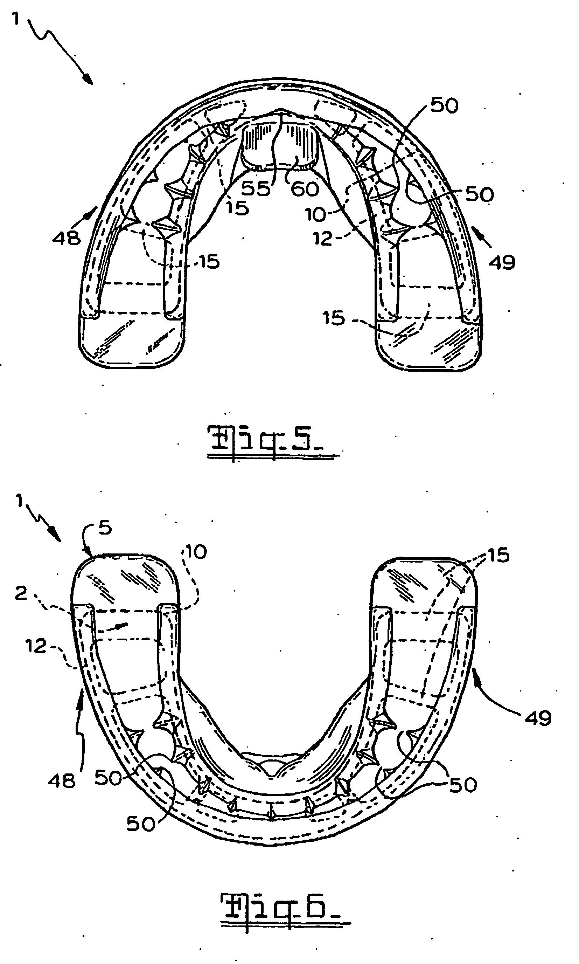

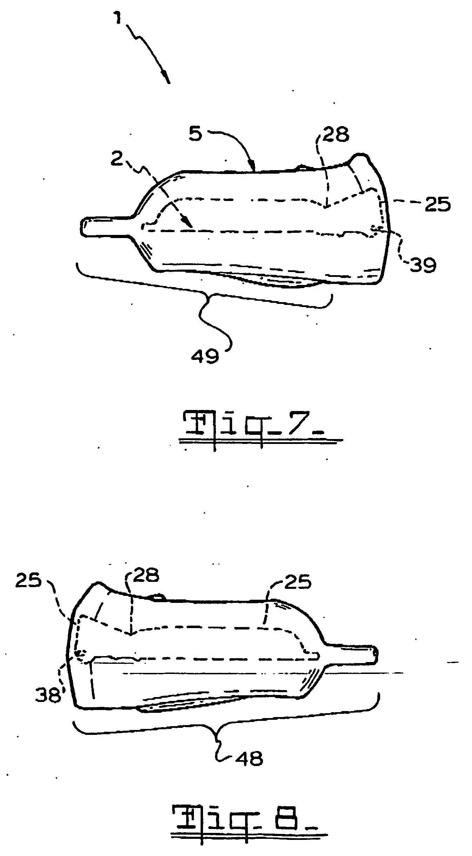

In Figs 1 to 9 reference numeral 1 refers generally to an appliance that is an orthodontic trainer in accordance with the invention.

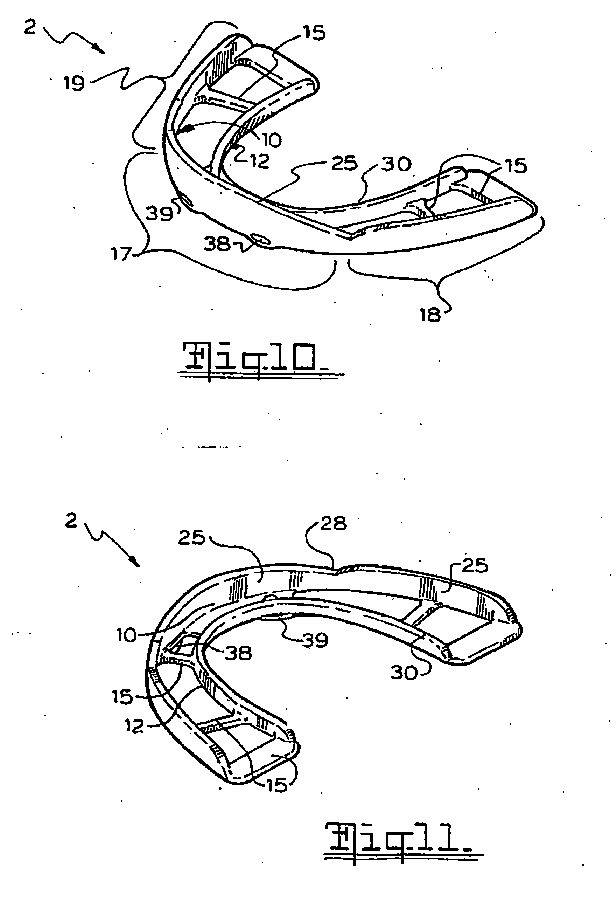

The orthodontic trainer 1 comprises generally a base member 2 having a generally U-shaped form corresponding to the outline of the jaw of a user. The base member 2 which is shown in Figs 10 and 11 on its own is indicated by hidden detail lines in Figs 1 to 9. The orthodontic trainer 1 further includes a teeth engagement member 5 made of silicone rubber that encloses and encases the base member 2. The teeth engaging member 5 occupies a substantially greater volume than the base member 2 and forms the body and shape of the trainer 1. It also defines all the operative surfaces that interact with and engage the teeth and gum tissues of the mouth of a user.

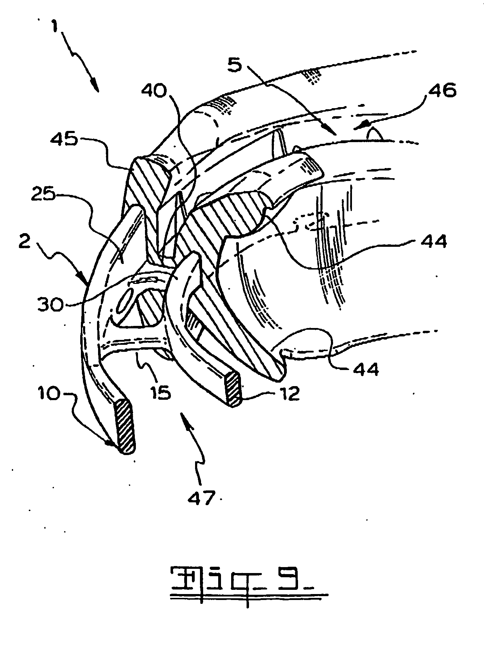

The base member 2 is shown partially clearly in Figs 9 to 11 and has a base support in the form of an open frame structure with curved outer and inner longitudinal frame members 10 and 12 joined at spaced intervals by transverse frame members 15

The base member comprises a frontal portion indicated generally by numeral 17, and left and right arm regions indicated generally by numerals 18 and 19.

In the illustrated embodiment the base member 2 has several transverse frame members 15. One of these is located proximate to the front central region 17 of the circular elements 10, 12. This transverse frame member 15 has a width of about 8-12mm and is considerably thicker than the other transverse frame members. The additional width serves to confer additional strength on this transverse frame member 15. Two further transverse frame members 15 are located adjacent the rear of the left and right trailing arm regions 18, 19 of the base member 2. These transverse frame members 15 have a width of 3-6mm. While they are thinner than the front transverse frame member they are thicker than the remaining transverse frame members.

The remaining transverse frame members are known as intermediate transverse frame members 15. In the illustrated embodiment there are two said transverse frame members on each arm region 18, 19. The transverse frame members have a width of 1-4mm, e.g. about 2mm.

Further the base member 2 also includes an outer flange 25 projecting up from the curved outer frame member 10 and extending along the full length of the curved outer frame member 10. The outer flange 25 projects up above the transverse frame members 15 of the open frame structure and the general plane of the open frame (hereinafter called the outer flange).

The portion of the flange 25 in the front central region 17 of the outer curved frame member 10 projects further than that portion extending up from the left and right arm regions 18 and 19. The flange portion in the front central region 17 may have a maximum height of 6-8mm. The flange portions on the left and right arm regions of the outer frame member 10 may have a maximum height of 5-7mm.

The outer flange 25 defines a low point 28 on each side intermediate the central front portion 17 and the left and right hand arm regions 18, 19. The low point is positioned at the point where the eye teeth or canine teeth of a user are located. The reason for this is that the canine teeth are sometimes positioned laterally outward of the other teeth and if the flange was higher in this area the trainer might not fit over a user's teeth. Applicant has found that by reducing the height of the outer flange 25 at this point on each side the trainer can be fitted to most mouths without this problem occurring.

The curved inner frame member 12 of the base member 2 also has a flange 30 extending up from the open frame (hereinafter called the inner flange). The inner flange 30 projects up above the open frame a distance of 1-3mm, e.g. about 2mm. It is thus far less upstanding and prominent than the outer flange 25.

The curved outer and inner frame members 10, 12 and the outer and inner flanges. 25 and 30 together apply a correcting force to any misaligned teeth in the upper and lower arches of a user and thereby actively promote correct positioning of the upper teeth. In addition it provides further rigidity or stiffness and structural strength to the base member 2.

The base member 2 in the drawings does not have a flange depending or extending down below the open frame of the base member 2 to any appreciable extent. Applicant has found that the upper flange provides the base member 2 with the necessary level of torsional rigidity and stiffness and a lower flange is not necessary. However it is to be clearly understood that a downwardly depending flange is considered to fall within the scope of this invention. For example, a downwardly depending flange would further increase the strength of the base member.

The base member 2 defines a pair of openings 38, 39 in its central front region 17. The openings 38, 39 are formed in both the inner and outer curved frame members 12, 10 of the base member 2. These are located on left and right sides of the front region 17 of the base member 2. The openings are formed by integral bracket or loop formations that depend down below the base member 2.

The openings 38, 39 cooperate with corresponding openings in the teeth engaging member 5 as will be described in more detail below.

The base member 2 is made of a rigid plastics material that does not melt or soften when exposed to temperatures below about 300°C. In the illustrated embodiment the base member is made of a polyamide material that is nylon. Nylon does not soften or melt when it comes into contact with molten silicone when the silicone is injection moulded onto the nylon. In addition it is hard and has been found to have an appropriate level of rigidity. At the same time it permits some movement of the left and right arm regions towards and away from each other and some twisting of the left and right arm regions relative to each other.

Nylon is a generic name of any long chain synthetic polymeric amide which has recurring amide groups as an integral part of the main polymer chain. The polymer is linear and as such is suited to being formed into a filament although this need not be the case. One of the properties of nylon that makes it suitable for this application is that it is capable of withstanding particularly high temperatures. As a result it does not soften or deform when it is brought into contact with molten silicon at high temperatures.

Applicant has obtained nylon from Shinko Chemical Company based in Taipei, Taiwan. The table below indicates the different grades of Nylon 66 supplied by this company.

| | | | | | | | |
|---|---|---|---|---|---|---|---|
| **TENSILE STRENGTH** | KG/cm² | 800 | 900 | 1700 | 1900 | 840 | 1150 |
| **ELONGATION** | % | 55 | 10 | 7.1 | 2 | 4 | 4.5 |
| **FLEXURAL STRENGTH** | KG/cm² | 1000 | 1350 | 2300 | 2600 | 1200 | 1700 |
| **FLEXURAL MODULUS** | KG/cm² | 28000 | 35000 | 80000 | 108000 | 31000 | 72000 |
| **IZOD IMPACT STRENGTH** | KG-cm/cm | 13 | 8.5 | 11 | 9 | 7.3 | 7 |
| **ROCK WELL HARDNESS** | R-SCALE | 118 | 119 | 120 | 120 | 118 | 119 |
| **MELTING POINT** | °C | 260 | 260 | 255 | 260 | 260 | 260 |
| **M.D.T (18.6KG/cm²)** | °C | 66 | 200 | 238 | 240 | 73 | 248 |
| **M.D.T (4.6KG/cm²)** | °C | 230 | 240 | 255 | 255 | 230 | 245 |
| **ASH CONTENT** | W1% | - | 13 | 33 | 45 | - | 25 |
| **MOLD SHRINKAGE** | | 1.7-1.8 | 0.3-0.5 | 0.2-0.4 | 0.2-0.3 | 1.0-1.3 | 0.3-0.5 |
| | | 1.3-1.4 | 0.8-1.0 | 0.7-1.0 | 0.3-0.5 | 0.7-1.0 | 0.7-1.0 |
| **M.F.** | g/10min | 55 | 20 | 13 | 10 | 43 | 20 |
| **SP Gr** | g/cm² | 1.1 | 1.2 | 1.35 | 1.46 | 1.16 | 1.38 |

The Applicant has utilised a grade of Nylon 66 known as Nylon66 6212GA for the appliances he has made. This material has the following properties:

| | |
|---|---|
| Tensile strength | 900 |
| Flexural Strength | 1350 |
| Rock Well Hardness | 119 |

Nylon can also be obtained from a number of other chemical suppliers including E I. Du Pont Nemours Chemical Company (DuPont) based in Delaware in the USA. Applicant believes that nylon obtained from Du Pont will also work satisfactorily. This material is a staple commodity and could be obtained from a large number of nylon suppliers around the world. Applicant envisages that the invention would be able to be practised equally well no matter which supplier the nylon was sourced from.

The teeth engaging member 5 comprises a central web 40 that broadly surrounds and encases the open frame portion of the base member 2. It fills in the space between the cross elements 15 and also forms a layer of some thickness over the open frame. The teeth engaging member 5 also has inner and outer flanges 44, 45 extending both upwardly and downwardly both sides of the web 40. These flanges 44, 45 together with the web 40 form upper and lower channels 46, 47 within which the upper and lower teeth of a user are received. Like the base member 2 the teeth engaging member 5 may comprise a central front region and left and right arm regions 48 and 49.

The teeth engaging member is made of a polymeric compound containing silicon that is a silicone rubber that is of medical grade. This rubber is a staple commodity and can be bought from a number of well known chemical companies. For example, Applicant is aware that it can be bought from Du Pont Chemical Company based in Delaware in the USA.

Applicant has sourced a suitable silicone rubber from a Japanese chemical company by the name of Shin-Etsu Chemical Co Ltd based at 6-1, 2 Chome, Ohtemachi, Chiyodaku, Tokyo, Japan. The material specification data sheet provided by Shin-Etsu for this material is provided below.

### MATERIAL SPECIFICATION DATA SHEET

| SHIN-ETSU ® TWO-COMPONENT SILICONE RUBBER COMPOUND | | | Transparent High Strength | | |
|---|---|---|---|---|---|
| Typical Properties | | Units 50 | KE-1950-50 (A-B) | KE-1950-60 (A-B) | KE-1950-70 (A-B) |
| Viscosity in mPa.s | | | 680 | 730 | 750 |
| (P) Brookfield-type rotational viscometer | | | (6800) | (7300) | (7500) |
| Specific Gravity at 25°C (77°F) g/cm³ | | | 1.13 | 1.14 | 1.15 |
| Mixing Ratio A:B | | | 1:1 | 1:1 | 1:1 |
| Hardness | JIS-A | | 50 | 58 | 68 |
| Tensile Strength | JIS-6301 | Mpa | 9.3 | 7.8 | 7.8 |
| Elongation at break | JIS-6301 | % | 55 | 380 | 350 |
| Tear Strength | JIS-6301 | kN/m | 44.1 | 43.1 | 49 |
| Compression set | 22h/150°C | (%) | 28 | 22 | 50 |
| Linear Shrinkage | JIS-6301 | (%) | 2 | 1.9 | 2.1 |
| Volume Resistivity Comments | | Ω-m | 10T | 10T | 10T |

The grade of the silicone that the Applicant has used the most frequently for the appliance is KE-1950-70, which is the hardest grade.

Another supplier of silicone rubber is the Bayer Chemical Company based in Leverkusen, Germany. Bayer supplies a liquid silicone rubber LSR 2050 that is non toxic and suitable as a medical grade material. It is a two component rubber with each component packed in a separate container. These two components are then pumped into a static mixer and mixed thoroughly and then- injected into the injection mould die.

The trainer also includes teeth positioning formations 50 in each of the inner and outer flanges 44, 45 of the teeth engaging member 5. The positioning formations 50 are for directing and biasing individual teeth into their correct position on the arch of the user.

Each said teeth positioning formation 50 comprises protrusions projecting out from each of the inner and outer flanges 44, 45 into the space defined by the upper and lower channels. Each of the protrusions of the teeth positioning formations 50 may be wedge shaped when viewed in cross section tapering inwardly from both sides to a point at the desired position between adjacent teeth.

Further there may be teeth positioning formations 50 in each of the upper and lower channels of the teeth engaging member 5.

In the illustrated embodiment there are teeth positioning formations 50 for locating the ten teeth of a user nearest the front of the arch on the upper jaw. Similarly the tooth engaging formations may also locate the ten forward teeth on the lower jaw of a user.

The orthodontic trainer may also include a notch or cut-out 55 in the midline upper surface of the outer flange 45 of the teeth engaging member 5. It also includes a smaller midline notch or cut-out 57 in the lower surface of the outer flange 45. The notches remove material from the teeth engaging member on the mid line so that it does not come into contact with soft tissue in this area. There is a tendon that extends across this area and it is more comfortable for a user if the teeth engaging member 5 does not come into contact with this tendon.

The orthodontic trainer 1 also has a tongue tab 60 for positioning the tongue of a user in an exact central position. The tongue tab is formed in the inner flange 44 of the member 5 upwardly of the web 40. This assists in improving the oral habits of a user and particularly in avoiding tongue thrusting. The spaces on either side of the tab 60 perform the important function of permitting inward and outward adjustment of the arms of the trainer 1 to accommodate different arch sizes in different users. This enables two sizes of appliances with two different arch sizes to be capable of being fitted to most patients. There is also a small notch formed in the lower edge of the inner flange, eg in a central position.

Further the web 40 of the teeth engaging member 5, eg upper and lower faces thereof, tapers outwardly from the front region of the member 4 in a rear direction to the left and right trailing arm regions 48, 49. The effect of this is to progressively thicken the web 40 in a direction from the front to the rear of the teeth enraging member 5. This continues up to a point in the left and right trailing arm regions 48, 49 that is spaced forward of the rear ends of the arm regions. Thereafter the upper and lower faces of the engaging member 5 taper inwardly towards each other so as to progressively thin from said point to the rear of the teeth engaging member 5. In summary the web 40 can generally be described as having an asymmetric aerofoil shape on each arm region 48, 49 extending back from the front region. The aerofoil has a curved surface on its lower side and thus can be thought of as being inverted.

This aerofoil shape of the web 40 fills in the space between the upper and lower teeth of a user and supports the jaw. This enables the bottom jaw to assume its anatomically correct position in relation to the upper jaw and this has relaxation and muscular benefits to the user.

The teeth engaging member 5 also has passages defined therein in the same place as the apertures 38, 39 of the base member 2. These passages are continuous and open at both ends. The openings 38 and 39 in the base member increases the surface area of the teeth engaging member 5 in contact with the base member 2. This is advantageous because it also assists in holding the teeth engaging member on the base member and resisting delamination.

The orthodontic trainer is manufactured as follows. First the base member 2 is injection moulded from nylon in a first injection moulding step. The teeth engaging member 5 is then moulded around and over the base member 2 and encases the base member 2 in a second moulding step. The formed orthodontic trainer 1 can then be removed from the die.

The cycle times for each of the moulding steps may be about 15 seconds. The cycle time for the silicone rubber moulding will be longer than that for the nylon base member. Generally the moulded pieces are allowed to cool passively. However the silicone rubber can be actively cooled once it has been moulded. Generally the members are removed from the die once the moulded material has had an opportunity to cool sufficiently.

The molten silicone is introduced to the die at a very high temperature, eg at least 300°C. Therefore the moulded base member has to be able to withstand this temperature without softening. Nylon is capable of withstanding the temperatures of injection of the silicone and thus has been found to be very suitable for this purpose. Further nylon has a high level of stiffness and local hardness while at the same time permitting some flexing of the arm regions of the trainer towards and away from each other.

The trainer may be moulded in two separate dies with the base member being moulded in a first die then being removed and placed in a second die where the teeth engaging member is moulded.

Alternatively the base member and the teeth engaging member may be moulded in the-same mould in a co injection process. That is the base member is moulded in a first step by an injection moulding process and then subsequent thereto the teeth engaging member is moulded onto the base member by a second moulding step. The base member does not have to be removed from the mould before the teeth engaging member is moulded onto it. The mould comprises two mould parts, one for each of the base member and the teeth engaging member, that are brought in sequence into operative positions in the moulding area.

In use the orthodontic trainer described above will typically be fitted by an orthodontist or a dentist. The trainers envisaged by the Applicant will have at least two different sizes of base member. Each of these sizes of base member will then have at least four different sizes of teeth engaging member with different sizes of teeth locating formations.

Accordingly the first step of the practitioner will be to choose an appropriate trainer from the different sizes of trainers and insert it into a patient's mouth. Generally a practitioner will choose a size of trainer after inspecting and measuring the arch and teeth of the user. However a trial and error procedure could also be used whereby the practitioner tried each of the different sizes of appliance and then selected the one that fitted the best.

Once fitted to the patient's mouth the soft silicone rubber of the teeth engaging member bears against the gum and teeth of the user. The skeletal base member provides the underlying strength to preserve the shape and form of the arch defined by the trainer and also urges the teeth into a desired position.

The inner and outer flanges of the teeth engaging member bear against the teeth. While the silicone is soft and has some ability to conform to the mouth of a user it is also resilient and thus when it is deformed by the teeth it applies a return force against the teeth of the user. This force tends to align individual teeth so that they do not protrude or retract. The underlying rigidity of the base member maintains the shape of the arch so that it forms a smooth curved or parabolic shape when viewed in plan view. Thus a narrow arch on a user is urged to widen by the resilient biasing force of the deformed base member. This is analogous to active spring energy. The biasing force is applied against the teeth tending to widen out the arch. The teeth positioning formations also encourage the individual teeth to take up their preferred position by along the length of the line of the arch. The application of force to move teeth is standard practice in orthodontics.

The orthodontic trainer receives both the upper and lower arches and teeth of a user and thus is not suitable for use during every day living. For example a user could not talk or eat while they were wearing the trainer. The trainer will typically be worn by a user for a few hours each night. It is also advantageous if the trainer is also worn for a few hours in the day time when this is possible. Over time through the application of force to the appropriate face of the teeth it will tend to move the teeth towards their desired positions. The physiological mechanism by which tooth movement is accomplished is well understood by the dental and orthodontic community and will not be described in this specification.

In another embodiment of the invention that has not been illustrated in the drawings the base member is made out of nylon and the teeth engaging member is made of polyvinylchloride (PVC).

PVC resin is a staple commodity in the chemical industry and is available from a large number of chemical manufacturers around the world. Applicant has obtained PVC resin from IMPRODEX which is a division of Pacific Dunlop Limited based at 135 Racecourse Road, Flemington, VIC, Australia.

The specification for the product used by the Applicant is HYCO 4016-89 PVC compound. This is a clear extrusion grade PVC compound for applications requiring good clarity and low toxicity and is suitable for food contact use.

The properties of this PVC grade are as follows:

| SPECIAL PROPERTIES | |
|---|---|
| Shore A Hardness (ASTM 2240) Instantaneous | 79 |
| Shore A Hardness (ASTM 2240) 10 second delay | 71 |
| Specific Gravity | 1.22 |
| Tensile Strength | 17.7MPa |
| Elongation at Break | 400% |

A trainer made of these materials and having the same structural features and form as the silicone rubber trainer described above is manufactured by a similar two step moulding process. The base member is moulded of nylon in a first step. Thereafter the teeth engaging member is manufactured of PVC in a second injection moulding step.

An advantage of using PVC instead of silicone rubber is that it does not require as high an injection temperature. This simplifies the moulding equipment that is used. It also reduces the temperature that the base member is required to withstand when the teeth engaging member is moulded onto it. This then opens up the possibility of using materials other than nylon for the base member. Applicant envisages that other materials such as addition polymers, eg such as polyethylene, and polypropylene, and condensation polymers such as polyurethane and polycarbonate and a thermoplastic elastomer such as santoprene may be used. Further other thermoplastic material may also be suitable.

In use this trainer is used in the same way as the trainer described above. The PVC has similar properties to silicone and the other polymeric materials if they are used have similar properties in use as the nylon.

The nylon provides sufficient resilience and spring force to enable the appliance to be fitted to most user's with some degree of comfort without any moulding being carried out. Further the softness of the silicone rubber also assists the guard to be fitted to a user with a reasonable degree of comfort notwithstanding that it has not been moulded.

An advantage of the appliance described above with reference to the drawings is that it can apply a force that is strong enough to widen the arch and reposition teeth. The force that is applied to the teeth is comparable to that achieved by other orthodontic appliances. The repositioning force is due to the underlying strength of the nylon base member. The base member has the effect of applying a force to misaligned teeth, be it protruding or retracting, and this force tends to return them to the arch form or the arch line.

The flange on the outer curved frame member of the base member assists with the realignment of teeth and widening of the arch of the user. The inner and outer longitudinal frame members of the base member including the flanges have the effect of pushing the teeth forward when they are retruded or misaligned. Similarly if the tooth is rotated, with the distal edge protruding and the mesial edge retruded, then the inner and outer flanges also apply a force to encourage rotational realignment to the correct position. Yet further if the arch form is narrow, the inner and outer frame members are designed with a wide arch form and are resilient, and thus urge the teeth outwardly to widen the arch form.

Thus the trainer does not simply align adjacent teeth with each other so that they do not protrude. Rather it tends to widen the arch if it is narrower than the ideal arch width. It also correctly positions the individual teeth at the correct point along the line of the arch. This way the line between the two front teeth is positioned on a mid-line of the arch. This is an important requirement for satisfactory cosmetic orthodontics.

A further advantage of the orthodontic trainer described above is that the silicone rubber is a very soft material. It therefore has the ability to conform to the contours of a user's teeth and gums without applying too much local pressure to the teeth and gums. As such the trainer is not unduly harsh on a user's mouth. It does not tend to injure the soft tissues in a patient's mouth and also can be worn with a reasonable degree of comfort.

Yet further the trainer can be manufactured in a moulding operation in large quantities. The appliance is injection moulded in a two step moulding operation. This will enable it to be produced at reasonable cost. It does not need to be custom moulded for each individual user. This enables it to be supplied to the market at a reasonable cost.

A further advantage of the orthodontic trainer described above is that it has locating formations for positioning individual teeth of a user in an optimum position. This positioning of individual teeth on the parabolic curve of the arch with a mass manufactured trainer is a significant advance.

Yet further the orthodontic trainer has a tab for correctly positioning the tongue and spaces on either side of the tab to permit the arms of the trainer to be opened or closed to some extent.

It will of course be realised that the above has been given only by way of illustrative example of the invention and that all such modifications and variations thereto as would be apparent to persons skilled in the art are deemed to fall within the scope of the invention as claimed.

## Claims

1. An oral appliance that is an orthodontic trainer (1), comprising:
a base member (2) having a generally U-shaped form having left and right arms (18, 19), corresponding to the outline of the jaw of a user, the base member (2) being made of a polymeric material that is resilient and is capable of some bending when opposing arms (18, 19) of the base member (2) are pulled towards and away from each other; and
a continuous teething engaging member (5) made of a material that is softer than the material of the base member (2) that encloses and encases at least part of the base member (2) and defines at least one of upper and lower teeth channels (46, 47) within which the associated arch and teeth of the user can be received,
wherein the base member (2) is designed with a wide arch so that when fitted to a user having a narrow arch, the arch is urged to widen by the resilient force of the deformed base member (2).

2. An oral appliance according to claim 1, wherein the teeth engaging member (5) is made of a polymeric material.

3. An oral appliance according to claim 2, wherein the polymeric material of the teeth engaging member (5) is silicone rubber.

4. An oral appliance according to claim 2, wherein the polymeric material of the teeth engaging member (5) is an addition polymer which is polyvinyl chloride (PVC).

5. An oral appliance according to any one of claims 1 to 4, wherein the polymeric material from which the base member (2) is formed is a polyamide material that is formed either by a condensation polymerisation of amide monomers or a ring opening polymerisation of caprolactam.

6. An oral appliance according to any of claims 1 to 4, wherein the polymeric material of the base member (2) is an addition polymer including polyethylene or polypropylene or a condensation polymer including polyurethane or polycarbonate or a thermoplastic elastomer that is santoprene.

7. An oral appliance according to claim 1, wherein the base member (2) is made of polyamide material and the teeth engaging member (5) is made of silicone rubber.

8. An oral appliance according to any one of claims 1 to 7, wherein the base member (2) is in the form of an open frame structure.

9. An oral appliance according to claim 8, wherein the open frame structure comprises curved inner and outer longitudinal frame members (12, 10) that are interconnected at spaced intervals by transverse frame members (15) and wherein the longitudinal frame members (12, 10) and the transverse frame members (15) all lie broadly in the same plane.

10. An oral appliance according to claim 9, wherein the base member (2) further includes an outer teeth repositioning formation (25) of the outer longitudinal frame member (10).

11. An oral appliance according to claim 10, wherein the outer teeth repositioning formation (25) comprises an outer flange projecting up from the outer longitudinal frame member (10) above the height of the transverse frame members (15).

12. An oral appliance according to claim 11, wherein the base member (2) has a central front region (17) and the left and right arms (18, 19) extend rearward from the central front region (17) to left and right rear ends.

13. An oral appliance according to claim 12, wherein the outer flange (25) projects upwardly from the transverse frame members (15) along at least said central front region (17) whereby to extend over and across the upper front teeth of a user in use.

14. An oral appliance according to claim 13, wherein the central front region (17) of the outer flange (25) extends upwardly above an upper surface of the transverse frame members (15) by 2-10mm.

15. An oral appliance according to claim 13 or claim 14, wherein the outer flange (25) also projects upwardly above the transverse frame members (15) along the left and right arms (18, 19) of the base member.

16. An oral appliance according to claim 15, wherein the left and right arms (18, 19) of the outer flange (25) extend upwardly above the transverse frame members (15) of the open frame structure by 2-6mm whereby to extend over at least part of the molars of the user in use.

17. An oral appliance according to claim 15 or claim 16, wherein the outer flange (25) is interrupted or of reduced height at positions (28) on the left side intermediate the left arm (18) and the central front region (17) of the outer flange (25), and also on the right side intermediate the right arm (18) and the central front region (17) of the outer flange (25), said positions (28) on the left and right sides corresponding to the positions of the canine teeth of a user.

18. An oral appliance according to any one of claims 11 to 17, wherein the outer flange (25) is formed integrally with the curved outer longitudinal frame member (10).

19. An oral appliance according to any one of claims 10 to 17, wherein the base member (2) further includes an inner teeth repositioning formation (30) on the inner longitudinal frame member (12).

20. An oral appliance according to claim 19, wherein the inner teeth repositioning formation (30) comprises an inner flange projecting up from the inner frame member (12) above the height of the transverse frame members (15).

21. An oral appliance according to claim 20, wherein the inner flange (30) on the curved inner longitudinal frame member (12) extends a distance of about 1-3mm above an upper surface of the transverse frame members (15).

22. An oral appliance according to claim 20 or claim 21, wherein the inner flange (30) projects up from the transverse frame members (15) along the full length of the inner longitudinal frame member (12) and the inner flange (30) projects up to substantially the same height along its full length.

23. An oral appliance according to any one of claims 20 to 22, wherein the inner flange (30) is formed integrally with the curved inner longitudinal frame member (12).

24. An oral appliance according to any one of claims 8 to 23, wherein the base member (2) has a front said transverse frame member (15) in the central front region (17) of the base member (2), and also rear said transverse frame members (15) towards the rear of the left and right arms (18, 19) of the base member (2).

25. An oral appliance according to claim 24, wherein the base member (2) further includes intermediate transverse frame members (15) in between the rear transverse frame members (15) at the rear of the arms (18, 19) and the front transverse frame member (15).

26. An oral appliance according to claim 25, wherein the transverse frame member (15) in the central front region (17) of the base member (2) has a width of 5-15mm, and the rear transverse frame members (15) at the rear of each of the arms (18, 19) have a width of 2-10mm, and the intermediate transverse frame members (15) have a width of 1-4mm.

27. An oral appliance according to any one of claims 8 to 26, wherein the teeth engaging member (5) comprises a central web (40) having upper and lower surfaces that extends parallel to the plane of the open frame structure, and also inner and outer flanges (44, 45) that project transversely away from at least one of the upper and lower surfaces of the web (40) and define with the web (40) at least one channel (46, 47) within which the teeth of a user are received.

28. An oral appliance according to claim 27, wherein the open frame structure has upper and lower faces and inner and outer sides, and the teeth engaging member (5) encases the upper and lower faces of the open frame structure and also the inner and outer sides of the open frame structure.

29. An oral appliance according to claim 28, wherein the inner and outer flanges (44, 45) that project away from both the upper and lower surfaces of the web (40) define both upper and lower channels (46, 47) within which respectively the upper and lower rows of teeth of a user can be received.

30. An oral appliance according to claim 29, wherein the teeth engaging member (5) extends fully around and over the base member and completely encases the full surface of the base member.

31. An oral appliance according to claim 30, wherein the teeth engaging member (5) covers an outer surface of the outer flange (45) with a layer of material that is substantially thinner than the layer of material covering an inside surface of the outer flange (45) whereby to assist in holding the base member (2) and teeth engaging member (5) together.

32. An oral appliance according to any one of claims 28 to 31, wherein the teeth engaging member (5) further includes one or more teeth positioning formations (50) in at least one of the channels (46, 47), each said positioning formation (50) assisting in positioning the teeth of a user that are adjacent to it in use.

33. An oral appliance according to claim 32, wherein each teeth positioning formation (50) on the teeth engaging member (5) comprises a wedge shaped protrusion extending inwardly from a said flange (44, 45) into a said channel (46, 47), and wherein the wedge shaped protrusion is integrally moulded with the teeth engaging member (5).

## Patentansprüche

1. Orale Vorrichtung, die ein kieferorthopädisches Trainingsgerät (1) ist, umfassend:
ein Basisteil (2) mit einer insgesamt U-förmigen Gestalt, das, entsprechend der Kontur des Kiefers eines Benutzers, linke und rechte Arme (18, 19) besitzt, wobei das Basisteil (2) aus Polymermaterial hergestellt ist, das nachgiebig und zu einem gewissen Verbiegen imstande ist, wenn die gegenüberliegenden Arme (18, 19) des Basisteils (2) zu-und auseinandergezogen werden; und
ein durchgehendes Zähne-Eingriffsteil (5), das aus einem Material hergestellt ist, das weicher ist als das Material des Basisteils (2), mindestens einen Teil des Basisteils (2) einschließt und umgibt sowie mindestens einen des oberen und unteren Zähnekanals (46, 47) definiert, innerhalb dessen der zugeordnete Gaumenbogen und die zugeordneten Zähne des Benutzers aufgenommen werden können,
wobei das Basisteil (2) mit einem weiten Gaumenbogen ausgelegt ist, so dass, wenn sie an einen Benutzer angepasst wird, der einen engen Gaumenbogen hat, der Gaumenbogen zwangsweise durch die Federkraft des verformten Basisteils (2) erweitert wird.

2. Orale Vorrichtung nach Anspruch 1, wobei das Zähne-Eingriffsteil (5) aus einem Polymermaterial hergestellt ist.

3. Orale Vorrichtung nach Anspruch 2, wobei das Polymermaterial des Zähne-Eingriffsteils (5) Silikongummi ist.

4. Orale Vorrichtung nach Anspruch 2, wobei das Polymermaterial des Zähne-Eingriffsteils (5) ein Additionspolymer ist, das Polyvinylchlorid (PVC) ist.

5. Orale Vorrichtung nach irgendeinem der Ansprüche 1 bis 4, wobei das Polymermaterial, woraus das Basisteil (2) geformt ist, ein Polyamidmaterial ist, das entweder durch Kondensationspolymerisation von Amid-Monomeren oder eine ringöffnende Polymerisation von Caprolactam gebildet ist.

6. Orale Vorrichtung nach irgendeinem der Ansprüche 1 bis 4, wobei das Polymermaterial des Basisteils (2) ein Additionspolymer ist, das Polyethylen oder Polypropylen umfasst oder ein Kondensationspolymer ist, das Polyurethan umfasst, oder Polycarbonat oder ein thermoplastisches Elastomer ist, das Santopren ist.

7. Orale Vorrichtung nach Anspruch 1, wobei das Basisteil (2) aus Polyamidmaterial hergestellt ist, und das Zähne-Eingriffsteil (5) aus Silikongummi hergestellt ist.

8. Orale Vorrichtung nach irgendeinem der Ansprüche 1 bis 7, wobei das Basisteil (2) in Form einer offenen Rahmenstruktur vorliegt.

9. Orale Vorrichtung nach Anspruch 8, wobei die offene Rahmenstruktur gekrümmte innere und äußere Rahmen-Längsteile (12, 10) umfasst, die an beabstandeten Intervallen durch Rahmen-Querteile (15) miteinander verbunden sind, und wobei die Rahmen-Längsteile (12, 10) und die Rahmen-Querteile (15) alle etwa in derselben Ebene liegen.

10. Orale Vorrichtung nach Anspruch 9, wobei das Basisteil (2) ferner eine äußere Zahn-Repositionierungsausbildung (25) des äußeren Rahmen-Längsteils (10) umfasst.

11. Orale Vorrichtung nach Anspruch 10, wobei die äußere Zahn-Repositionierungsausbildung (25) einen äußeren Flansch umfasst, der vom äußeren Rahmen-Längsteil (10) aus über die Höhe der Rahmen-Querteile (15) hinaus vorspringt.

12. Orale Vorrichtung nach Anspruch 11, wobei das Basisteil (2) einen mittleren Frontbereich (17) aufweist und sich die linken und rechten Arme (18, 19) vom mittleren Frontbereich (17) aus rückwärts zu linken und rechten, rückwärtigen Enden erstrecken.

13. Orale Vorrichtung nach Anspruch 12, wobei der äußere Flansch (25) nach oben von den Rahmen-Querteilen (15) aus längs mindestens des genannten mittleren Frontbereiches (17) vorspringt, um sich hierdurch im Gebrauch über die oberen Frontzähnen eines Benutzers und quer zu diesen zu erstrecken.

14. Orale Vorrichtung nach Anspruch 13, worin der mittlere Frontbereich (17) des äußeren Flansches (25) sich um 2-10mm nach oben bis über eine obere Oberfläche der Rahmen-Querteile (15) hinaus erstreckt.

15. Orale Vorrichtung nach Anspruch 13 oder Anspruch 14, worin der äußere Flansch (25) auch nach oben bis über die Rahmen-Querteile (15) längs der linken und rechten Arme (18, 19) des Basisteils vorspringt,

16. Orale Vorrichtung nach Anspruch 15, wobei sich die linken und rechten Arme (18, 19) des äußeren Flansches (25) nach oben um 2-6mm bis über die Rahmen-Querteile (15) der offenen Rahmenstruktur erstrecken, um sich hierdurch im Gebrauch über mindestens einen Teil der Backenzähne des Benutzers zu erstrecken,

17. Orale Vorrichtung nach Anspruch 15 oder Anspruch 16, wobei der äußere Flansch (25) an Stellen (28) auf der linken Seite zwischen dem linken Arm (18) und dem mittleren Frontbereich (17) des äußeren Flansches (25) und auch auf der rechten Seite zwischen dem rechten Arm (18) und dem mittleren Frontbereich (17) des äußeren Flansches (25) unterbrochen oder von verringerter Höhe ist, wobei die genannten Positionen (28) auf der linken und rechten Seite den Positionen der Eckzähne eines Benutzers entsprechen,

18. Orale Vorrichtung nach irgendeinem der Ansprüche 11 bis 17, wobei der äußere Flansch (25) einstückig mit dem gekrümmten, äußeren Rahmen-Längsteil (10) geformt ist.

19. Orale Vorrichtung nach irgendeinem der Ansprüche 10 bis 17, wobei das Basisteil (2) ferner eine innere Zahn-Repositionierungsausbildung (30) auf dem inneren Rahmen-Längsteil (12) umfasst.

20. Orale Vorrichtung nach Anspruch 19, wobei die innere Zahn-Repositionierungsausbildung (30) einen inneren Flansch umfasst, der vom inneren Rahmenteil (12) aus nach oben bis über die Höhe der Rahmen-Querteile (15) vorspringt.

21. Orale Vorrichtung nach Anspruch 20, wobei sich der innere Flansch (30) auf dem gekrümmten, inneren Rahmen-Längsteil (12) um einen Abstand von etwa 1-3mm bis über eine obere Oberfläche der Rahmen-Querteile (15) erstreckt.

22. Orale Vorrichtung nach Anspruch 20 oder Anspruch 21, wobei der innere Flansch (30) von den Rahmen-Querteilen (15) aus nach oben längs der vollen Länge des inneren Rahmen-Längsteils (12) hinaus vorspringt, und der innere Flansch (30) bis zur im Wesentlichen selben Höhe längs seiner vollen Länge vorspringt

23. Orale Vorrichtung nach irgendeinem der Ansprüche 20 bis 22, wobei der innere Flansch (30) einstückig mit dem gekrümmten, inneren Rahmen-Längsteil (12) ausgebildet ist.

24. Orale Vorrichtung nach irgendeinem der Ansprüche 8 bis 23, wobei das Basisteil (2) eine Front des genannten Rahmen-Querteils (15) im mittleren Frontbereich (17) des Basisteils (2), und auch eine Rückseite der genannten Rahmen-Querteile (15) zur Rückseite der linken und rechten Arme (18, 19) des Basisteils (2) hin, aufweist.

25. Orale Vorrichtung nach Anspruch 24, wobei das Basisteil (2) ferner Zwischen-Rahmen-Querteile (15) zwischen den hinteren Rahmen-Querteilen (15) an der Rückseite der Arme (18, 19) und dem vorderen Rahmen-Querteil (15) umfasst.

26. Orale Vorrichtung nach Anspruch 25, wobei das Rahmen-Querteil (15) im mittleren Frontbereich (17) des Basisteils (2) eine Breite von 5-15mm umfasst, die hinteren Rahmen-Querteile (15) an der Rückseite eines jeden der Arme (18, 19) eine Breite von 2-10mm umfassen, und die Zwischen-Rahmen-Querteile (15) eine Breite von 1-4mm umfassen.

27. Orale Vorrichtung nach irgendeinem der Ansprüche 8 bis 26, wobei das Zähne-Eingriffsteil (5) einen mittleren Steg (40) umfasst, der obere und unteren Oberflächen aufweist, die sich parallel zur Ebene der offenen Rahmenstruktur erstrecken, und ebenfalls innere und äußere Flansche (44, 45), die quer von mindestens einer der unteren und oberen Oberflächen des Steges (40) vorspringen und mit dem Steg (40) mindestens einen Kanal (46, 47) definieren, innerhalb dessen die Zähne eines Benutzers aufgenommen werden.

28. Orale Vorrichtung nach Anspruch 27, wobei die offene Rahmenstruktur obere und untere Flächen sowie innere und äußere Seiten umfasst, und das Zähne-Eingriffsteil (5) die oberen und unteren Flächen der offenen Rahmenstruktur und auch die inneren und äußeren Seiten der offenen Rahmenstruktur einschließt.

29. Orale Vorrichtung nach Anspruch 28, wobei die inneren und äußeren Flansche (44, 45), die von den oberen und unteren Oberflächen des Steges (40) aus vorspringen, beide obere und untere Kanäle (46, 47) definieren, innerhalb derer jeweils die obere und untere Reihe von Zähnen eines Benutzers aufgenommen werden können.

30. Orale Vorrichtung nach Anspruch 29, wobei sich das Zähne-Eingriffsteil (5) voll um das Basisteil herum und über dieses hinaus erstreckt und vollständig die volle Oberfläche des Basisteils einschließt.

31. Orale Vorrichtung nach Anspruch 30, wobei das Zähne-Eingriffsteil (5) eine Außenoberfläche des äußeren Flansches (45) mit einer Materialschicht abdeckt, die wesentlich dünner ist als die Materialschicht, die eine Innenoberfläche des äußeren Flansches (45) abdeckt, um hierdurch zum Zusammenhalten von Basisteil (2) und Zähne-Eingriffsteil (5) beizutragen.

32. Orale Vorrichtung nach irgendeinem der Ansprüche 28 bis 31, wobei das Zähne-Eingriffsteil (5) ferner eine oder mehrere Zähne-Positionierungsausbildungen (50) in mindestens einem der Kanäle (46, 47) umfasst, wobei jede genannte Positionierungsausbildung (50) dazu beiträgt, die Zähne eines Benutzers, die zu ihr benachbart sind, zu positionieren.

33. Orale Vorrichtung nach Anspruch 32, wobei jede Zähne-Positionierungsausbildung (50) am Zähne-Eingriffsteil (5) einen keilförmigen Vorsprung umfasst, der sich von einem genannten Flansch (44, 45) aus nach innen in einen genannten Kanal (46, 47) erstreckt, und wobei der keilförmige Vorsprung mit dem Zähne-Eingriffsteil (5) einstückig geformt ist.

## Revendications

1. Appareil buccal se présentant comme un correcteur orthodontique (1), comprenant :
un élément de socle (2) offrant une configuration générale en U présentant des ailes (18, 19) de gauche et de droite, correspondant au contour extérieur de la mâchoire d'un utilisateur, l'élément de socle (2) étant constitué d'un matériau polymère à détente élastique, apte à fléchir sensiblement lorsque les ailes opposées (18, 19) dudit élément de socle (2) sont rapprochées et éloignées l'une de l'autre par traction ; et
un élément continu (5) en contact avec les dents, constitué d'un matériau plus tendre que le matériau de l'élément de socle (2), qui enveloppe et emprisonne au moins une partie dudit élément de socle (2) et définit au moins l'une, parmi des gouttières dentaires supérieure et inférieure (46, 47) dans lesquelles peuvent prendre place l'arcade et les dents associées de l'utilisateur,
sachant que l'élément de socle (2) est pourvu d'une arcade large, de sorte que, lorsque implanté sur un utilisateur muni d'une arcade étroite, ladite arcade est contrainte à s'élargir sous l'action de la force de détente élastique dudit élément de socle (2) déformé.

2. Appareil buccal selon la revendication 1, dans lequel l'élément (5) en contact avec les dents est constitué d'un matériau polymère.

3. Appareil buccal selon la revendication 2, dans lequel le matériau polymère de l'élément (5) en contact avec les dents est du caoutchouc siliconé.

4. Appareil buccal selon la revendication 2, dans lequel le matériau polymère de l'élément (5) en contact avec les dents est un polymère d'adjonction se présentant comme du polychlorure de vinyle (PVC).

5. Appareil buccal selon l'une quelconque des revendications 1 à 4, dans lequel le matériau polymère, dont l'élément de socle (2) est constitué, est un matériau polyamide obtenu soit par polymérisation par condensation de monomères d'amide, soit par polymérisation d'un caprolactame par ouverture de cycle.

6. Appareil buccal selon l'une quelconque des revendications 1 à 4, dans lequel le matériau polymère de l'élément de socle (2) est un polymère d'adjonction incluant du polyéthylène ou du polypropylène, ou un polymère de condensation incluant du polyuréthane ou un polycarbonate, ou un élastomère thermoplastique se présentant comme du Santoprène.

7. Appareil buccal selon la revendication 1, dans lequel l'élément de socle (2) est constitué d'un matériau polyamide, et l'élément (5) en contact avec les dents est constitué de caoutchouc siliconé.

8. Appareil buccal selon l'une quelconque des revendications 1 à 7, dans lequel l'élément de socle (2) revêt la forme d'une structure d'ossature ouverte.

9. Appareil buccal selon la revendication 8, dans lequel la structure d'ossature ouverte comprend des éléments d'ossature longitudinaux (10, 12) intérieur et extérieur, de forme incurvée, qui sont reliés l'un à l'autre à intervalles par des éléments d'ossature transversaux (15) ; et dans lequel lesdits éléments d'ossature longitudinaux (10, 12) et éléments d'ossature transversaux (15) sont tous approximativement situés dans le même plan.

10. Appareil buccal selon la revendication 9, dans lequel l'élément de socle (2) comporte, en outre, un profil extérieur (25) de repositionnement dentaire qui est situé sur l'élément d'ossature longitudinal (10) extérieur.

11. Appareil buccal selon la revendication 10, dans lequel le profil extérieur (25) de repositionnement dentaire comprend une collerette extérieure faisant saillie vers le haut à partir de l'élément d'ossature longitudinal (10) extérieur, au-dessus de la hauteur des éléments d'ossature transversaux (15).

12. Appareil buccal selon la revendication 11, dans lequel l'élément de socle (2) présente une région antérieure centrale (17) et les ailes (18, 19) de gauche et de droite s'étendent vers l'arrière, à partir de ladite région antérieure centrale (17), jusqu'à des extrémités postérieures de gauche et de droite.

13. Appareil buccal selon la revendication 12, dans lequel la collerette extérieure (25) fait saillie vers le haut à partir des éléments d'ossature transversaux (15), au moins le long de ladite région antérieure centrale (17) de manière à s'étendre, en service, au-dessus et en travers des dents antéro-supérieures d'un utilisateur.

14. Appareil buccal selon la revendication 13, dans lequel la région antérieure centrale (17) de la collerette extérieure (25) s'étend vers le haut, de 2-10 mm, au-dessus d'une surface supérieure des éléments d'ossature transversaux (15).

15. Appareil buccal selon la revendication 13 ou la revendication 14, dans lequel la collerette extérieure (25) est pareillement en saillie vers le haut, au-dessus des éléments d'ossature transversaux (15), le long des ailes (18, 19) de gauche et de droite de l'élément de socle,

16. Appareil buccal selon la revendication 15, dans lequel les ailes (18, 19) de gauche et de droite de la collerette extérieure (25) s'étendent vers le haut, de 2-6 mm, au-dessus des éléments d'ossature transversaux (15) de la structure d'ossature ouverte de manière à s'étendre, en service, au-dessus d'au moins une partie des molaires de l'utilisateur.

17. Appareil buccal selon la revendication 15 ou la revendication 16, dans lequel la collerette extérieure (25) comporte un décrochement ou une hauteur réduite en des emplacements (28) situés du côté gauche, à mi-distance entre l'aile de gauche (18) et la région antérieure centrale (17) de ladite collerette extérieure (25), et également du côté droit, à mi-distance entre l'aile de droite (18) et ladite région antérieure centrale (17) de ladite collerette extérieure (25), lesdits emplacements (28), situés du côté gauche et du côté droit, correspondant aux emplacements des canines d'un utilisateur.

18. Appareil buccal selon l'une quelconque des revendications 11 à 17, dans lequel la collerette extérieure (25) est ménagée d'un seul tenant avec l'élément d'ossature longitudinal (10) extérieur, de forme incurvée.

19. Appareil buccal selon l'une quelconque des revendications 10 à 17, dans lequel l'élément de socle (2) offre, par ailleurs, un profil intérieur (30) de repositionnement dentaire qui se trouve sur l'élément d'ossature longitudinal (12) intérieur.

20. Appareil buccal selon la revendication 19, dans lequel le profil intérieur (30) de repositionnement dentaire comprend une collerette intérieure faisant saillie vers le haut à partir de l'élément d'ossature (12) intérieur, au-dessus de la hauteur des éléments d'ossature transversaux (15).

21. Appareil buccal selon la revendication 20, dans lequel la collerette intérieure (30) située sur l'élément d'ossature longitudinal (12) intérieur, de forme incurvée, s'étend d'une distance d'environ 1-3 mm au-dessus d'une surface supérieure des éléments d'ossature transversaux (15).

22. Appareil buccal selon la revendication 20 ou la revendication 21, dans lequel la collerette intérieure (30) est en saillie vers le haut à partir des éléments d'ossature transversaux (15), sur l'intégralité de la longueur de l'élément d'ossature longitudinal (12) intérieur, et ladite collerette intérieure (30) dépasse vers le haut sensiblement jusqu'à la même hauteur, sur l'intégralité de sa longueur.

23. Appareil buccal selon l'une quelconque des revendications 20 à 22, dans lequel la collerette intérieure (30) est ménagée d'un seul tenant avec l'élément d'ossature longitudinal (12) intérieur, de forme incurvée.

24. Appareil buccal selon l'une quelconque des revendications 8 à 23, dans lequel l'élément de socle (2) comprend un élément d'ossature transversal (15) antérieur, dans la région antérieure centrale (17) dudit élément de socle (2), et également des éléments d'ossature transversaux (15) postérieurs qui pointent vers l'arrière des ailes (18, 19) de gauche et de droite dudit élément de socle (2).

25. Appareil buccal selon la revendication 24, dans lequel l'élément de socle (2) comporte, en outre, des éléments d'ossature transversaux (15) intermédiaires qui sont interposés entre les éléments d'ossature transversaux (15) postérieurs, à l'arrière des ailes (18, 19), et l'élément d'ossature transversal (15) antérieur.

26. Appareil buccal selon la revendication 25, dans lequel l'élément d'ossature transversal (15), situé dans la région antérieure centrale (17) de l'élément de socle (2), possède une largeur de 5-15 mm, les éléments d'ossature transversaux (15) postérieurs, situés à l'arrière de chacune des ailes (18, 19), présentent une largeur de 2-10 mm, et les éléments d'ossature transversaux (15) intermédiaires offrent une largeur de 1-4 mm.

27. Appareil buccal selon l'une quelconque des revendications 8 à 26, dans lequel l'élément (5), en contact avec les dents, comprend une membrure centrale (40) présentant des surfaces supérieure et inférieure qui s'étendent parallèlement au plan de la structure d'ossature ouverte, et également des rebords (44, 45) intérieur et extérieur qui font saillie transversalement, en s'éloignant d'au moins l'une desdites surfaces supérieure et inférieure de la membrure (40) et définissent, avec ladite membrure (40), au moins une gouttière (46, 47) dans laquelle prennent place les dents d'un utilisateur.

28. Appareil buccal selon la revendication 27, dans lequel la structure d'ossature ouverte comporte des faces supérieure et inférieure, et des côtés intérieur et extérieur, et l'élément (5) en contact avec les dents emprisonne lesdites faces supérieure et inférieure de ladite structure d'ossature ouverte, et également lesdits côtés intérieur et extérieur de ladite structure d'ossature ouverte.

29. Appareil buccal selon la revendication 28, dans lequel les rebords (44, 45) intérieur et extérieur, faisant saillie en s'éloignant de l'une et l'autre des surfaces supérieure et inférieure de la membrure (40), définissent l'un et l'autre des gouttières (46, 47) supérieure et inférieure dans lesquelles peuvent prendre place, respectivement, les rangées de dents supérieure et inférieure d'un utilisateur.

30. Appareil buccal selon la revendication 29, dans lequel l'élément (5), en contact avec les dents, s'étend intégralement autour et au-dessus de l'élément de socle, et emprisonne la totalité de la surface dudit élément de socle.

31. Appareil buccal selon la revendication 30, dans lequel l'élément (5), en contact avec les dents, recouvre une surface extérieure de la collerette extérieure (45) par une couche de matériau qui est substantiellement plus mince que la couche de matériau recouvrant une surface intérieure de ladite collerette extérieure (45), favorisant ainsi la retenue réciproque de l'élément de socle (2) et dudit élément (5) en contact avec les dents.

32. Appareil buccal selon l'une quelconque des revendications 28 à 31, dans lequel l'élément (5), en contact avec les dents, inclut par ailleurs un ou plusieurs profil (s) (50) de positionnement dentaire, dans au moins l'une de gouttières (46, 47), chacun desdits profils de positionnement (50) favorisant, en service, un positionnement des dents d'un utilisateur qui lui sont adjacentes.

33. Appareil buccal selon la revendication 32, dans lequel chaque profil (50) de positionnement dentaire comprend, sur l'élément (5) en contact avec les dents, une protubérance cunéiforme qui s'étend vers l'intérieur, à partir de l'un desdits rebords (44, 45), et pénètre dans l'une desdites gouttières (46, 47) ; et dans lequel ladite protubérance cunéiforme est moulée d'une seule pièce avec ledit élément (5) en contact avec les dents.
